# EUROPEAN PATENT APPLICATION

(11) **EP 4 285 812 A1**
(43) Date of publication of application: **06.12.2023**
(21) Application number: 22176730.4
(22) Date of filing: 01.06.2022
(51) Int. Cl.: A61B 3/028, A61B 3/103

(54) **REFRACTOR AND METHOD FOR CONTROLLING A REFRACTOR**

(71) Applicant: Carl Zeiss Vision International GmbH, 73430 Aalen (DE)
(72) Inventor: BELZ, Carsten, 74594 Kressberg (DE)
(74) Representative: Sticht, Andreas

(57) **Abstract**

A refractor is provided, comprising a first refractor head (21A) and a second refractor head (21B), which are rotatable about respective axes (22A, 22B), and a controller configured to adjust angular positions of the first and second refractor heads (21A, 21B) about the respective axes (22A, 22B) on the basis of a distance (D) between a head (30) of the patient and a viewing target (31, 32) in a first direction perpendicular to the axes (22A, 22B). A first sensor (24A - 24C) is configured to measure at least part of the distance between the head and the viewing target. In this way, an automatic setting of the angular position may be performed.

## Description

The present application relates to refractors and methods for controlling such refractors.

A refractor is an ophthalmic testing device which may be used by eyecare professionals like doctors or opticians during an eye estimation to determine the refraction of one eye or both eyes of a patient.

Refractors as used herein are used at least for subjective refraction measurements, i.e. refraction measurements relying on a feedback from the patient. In this context, the refractor is also referred to as phoropter or as comprising a phoropter. The refractor typically includes two refractor heads (also referred to as phoropter heads), one refractor head for each eye. The refractor heads both include a respective optic, which may be adjusted in terms of refraction, for example in terms of sphere, cylinder, and axis, either by including a built-in adjustable optic (e.g. with movable and/or rotatable lenses) or by providing the possibility to insert different lenses.

The patient then looks through the refractor, each eye through the optic of one of the refractor heads, at an eye chart. For far refraction, the eye chart is placed at optical infinity, for example at 4m or more, like about 6 meters. For near refraction, the eye chart is placed closer to the patient, for example at about 40 cm. The eye chart may include any kinds of signs, symbols, text or the like. Generally, then the refraction of the refractor heads is changed, and the patient gives feedback when he can recognize the eye chart best. This process may be performed for each eye individually and/or for both eyes together.

Additionally, the refractor may include an automated refractor or autorefractor which performs an objective refraction measurement, for example by measuring reflexes in the eye, in any known manner, for example to provide a starting point for the subjective refraction measurement described above. Such refractors have been used for a long time and are well known in the art, such that the general operation will not be described in further detail here.

In some refractors, for example the Visionix VX160, the refractor heads may be adjusted either to a first angular position where the optical axes of the two refractor heads are parallel to each other, which is used for the far refraction measurement, and a second angular position where the optical axes run towards each other for near refraction measurements, to compensate the convergence of the eyes. However, as the eye chart for the near refraction measurements are not always at the same distance, the second angular position of the refractor heads is not always in the optimum position.

US 7 237 897 B2 discloses a refractor where an interpupillary distance between pupils of the eye of a patient and a distance to an eye chart is input to a control unit, and an angle of refractor heads is adjusted accordingly by the control unit. A similar device is known from JP 3 150 179 B2, where also refractor heads are adjusted based on a distance to an eye chart and an interpupillary distance of the patient.

However, in these devices an operator, for example optician or doctor, has to provide the interpupillary distance and the distance to the eye chart to a control unit, which requires some expertise in measuring these parameters.

Therefore, it is an object to provide a refractor and a corresponding method for controlling a refractor which simplifies this procedure.

According to a first aspect of the invention, a refractor (also referred to as phoropter) is provided, comprising
a first refractor head and a second refractor head, which are rotatable about respective axes, and
a controller configured to adjust angular positions of the first and second refractor heads about the respective axes on the basis of a distance between a head of the patient and a viewing target in a first direction perpendicular to the axes.

The refractor is characterized by a first sensor configured to measure at least part of the distance between the head and the viewing target.

By providing the first sensor and measuring at least part of the second distance therewith, the angular adjustment of the refractor heads may be performed in a fully automated manner, without input from an operator being necessary.

Terms used above will be explained in the following:
A refractor in the sense of the present application, also referred to as phoropter, is a device as explained in the background portion which includes first and second refractor heads, each including an optic, to determine the refraction of the patient, in particular by means of subjective refraction measurements. The refractor may also include an autorefractor to measure a starting point for the subjective refraction measurement by an objective refraction measurement.

"Measuring at least part of the distance between the head of the patient and the viewing target in the first direction" means that by measuring this part of the distance, the distance between the head, in particular a point about midway between the eyes, and the viewing target may be determined. For this, in some embodiments the complete distance may be measured by the first sensor. In other embodiments, the sensor may be configured to measure a distance between the viewing target and some reference point of the refractor, for example one or both of the refractor heads or some other element of the refractor.

In this respect, refractors usually include some reference elements for positioning the head, for example a chin rest or some element the forehead of the person is to abut against, such that in proper use, the head of the patient has a fixed spatial relationship to the refractor. It should be noted, that in case no such elements for the positioning of the head of the patients are provided, an operator of the refractor, for example optician or doctor, has to ensure a correct positioning of the head of the patient to ensure proper use. Proper use, as clear from the above explanations, refers to a usage of the refractor as intended. As in any device where some human interaction is required there is a possibility of maloperation, for example not aligning the head of the patient with the refractor correctly, which may lead to measurement errors.

Thus, in proper use the head of the patient has a fixed spatial relationship to the refractor. Therefore, by measuring the distance between the viewing target and a reference point of the refractor, also the distance between the head of the patient and the viewing target is known. To put it in other words, the part of the distance is an a priori unknown part of the distance between the head of the person and the viewing target, for example the above mentioned distance between a reference point of the refractor and the viewing target, and the remaining part of the distance is known in proper use of the refractor.

A viewing target refers to any element the patient should direct his or her gaze at during the refractive measurement. A typical viewing target may be an eye chart, also referred to as optotypes, having symbols, numbers, letters, words or text to read thereon. In other implementations, the viewing target may for example also include holograms for generating the impression of three-dimensional objects in space.

The controller may adjust the angular position of the first and second refractor heads in particular in a manner that the patient views the viewing target essentially along the optical axis of the respective refractor heads, i.e. looks "straight through" the refractor heads at the viewing target. Approximately in this case means that for an extended viewing target, this relationship strictly holds only for one point of the viewing target, and additionally the above mentioned measurements of the interpupillary distance and part of the distance may involve measurement tolerances. Furthermore, without the adaptation also to interpupillary distance as outlined below, the relationship only is strictly true for a particular interpupillary distance. For example, in this case the optical axes of the refractor had may be adjusted to intersect in the middle of the target.

The first sensor may include any conventional sensor for distance measurements. Examples include ultrasonic sensors, laser sensors, or other time of flight sensors.

The first sensor may be placed for example in the first or second refractor head, or in another part of the refractor which has a line of sight to the viewing target. In some embodiments, the viewing target in particular for near measurements may be part of the refractor, and in this case the first sensor may also be provided in the viewing target, to measure the distance either to the head of the patient or to a reference point of the refractor. In this case, for example inductive, optical, resistive, or capacitive sensors may be used. In some embodiments, the viewing target may be movable along a track, and the first sensor may be configured to measure a position of the viewing target on the rail, which in turn has a fixed spatial relationship to the refractor, thus measuring the part of the distance, for example as a sensor measuring the movement along the track or a sensor measuring movement (rotation) of a motor moving the viewing target. Therefore, various possibilities for placing the first sensor exist, and the first sensor is not limited to any particular placement.

The first refractor head and the second refractor head may be movable in a second direction perpendicular to the axes and perpendicular to the first direction, wherein the controller is further configured to adjust a position of the first and second refractor heads in the second direction based on an interpupillary distance of the patient and to adjust the angular position of the first and second refractor heads about the respective axes also based on the interpupillary distance.

In this way, the refractor may additionally be adapted to the interpupillary distance of the patient. In particular, the distance in the second direction between the first and second refractor heads may be adapted to the interpupillary distance, and the angular positions may be adapted accordingly such that the patient looks straight through the refractor heads for his/her interpupillary distance (see above explanations for looking straight through the refractor heads).

The interpupillary distance, usually abbreviated PD, is defined under item 5.29 of the DIN EN ISO 13666:2013-10 as the distance between the centers of the pupils when the eyes are fixating an object at an infinite distance in the straight ahead position.

The second direction mentioned above in proper use of the refractor is generally parallel to the direction in which the interpupillary distance is measured, or, in other words, parallel to a line between the pupils of the person when the interpupillary distance is measured.

The refractor may further comprise a second sensor configured to determine the interpupillary distance of the patient. In this way, the interpupillary distance may be determined automatically, such that a fully automated adjustment is possible. In other cases, the interpupillary distance may be known from previous measurements on the patient and therefore need not be determined by a second sensor.

The second sensor and the determination of the interpupillary distance may be implemented in any conventional manner, for example using a camera, which may be included in one or both of the refractor heads, for taking an image of an eye region and then determining the interpupillary distance based on the recorded images. For example, such a camera determining the interpupillary distance is implemented in the Visionix VX160 mentioned above, where a camera is placed below the refractor heads, and light is coupled out from the refractor heads to the camera.

After the setting of the angular position of the first and second refractor heads, and optionally also the positions in the second direction, refraction measurements may be performed as in conventional refractors.

According to the second aspect, a method for controlling a refractor including a first refractor head and a second refractor head is provided, comprising:
adjusting angular positions of the first and second refractor heads about respective axes on the basis of a distance between a head of a patient and a viewing target in a first direction.

The method is characterized by measuring at least part of the distance between the head of the patient and the viewing target using a first sensor.

The method may further comprise adjusting a position of the first refractor head and the second refractor head in a second direction perpendicular to the first direction and perpendicular to the axes based on an interpupillary distance of the patient, wherein adjusting the angular position of the first and second refractor heads about the respective axes is further based on the interpupillary distance.

The method may further comprise measuring the interpupillary distance of a patient using a second sensor.

The advantages and explanations given above for the refractor also apply to the methods.

The method may further include performing a refraction measurement after setting the position and angular of the first and second refractor heads.

According to a third aspect, a method for manufacturing a spectacle lens is provided, comprising:
measuring a refraction of a patient with the refractor described above or with the method described above, and
manufacturing a spectacle lens based on the refraction.

The manufacturing of the spectacle lens itself can be performed in any conventional manner, as known in the art.

Illustrative embodiments will now be described referring to the attached drawings.
Fig. 1 is a flowchart illustrating a method according to an embodiment.
Fig. 2 is a schematic diagram illustrating a refractor according to an embodiment.
Figures 3A to 3C show illustrative examples for further explaining the method of Fig. 1 and operation of the refractor of Fig. 2.

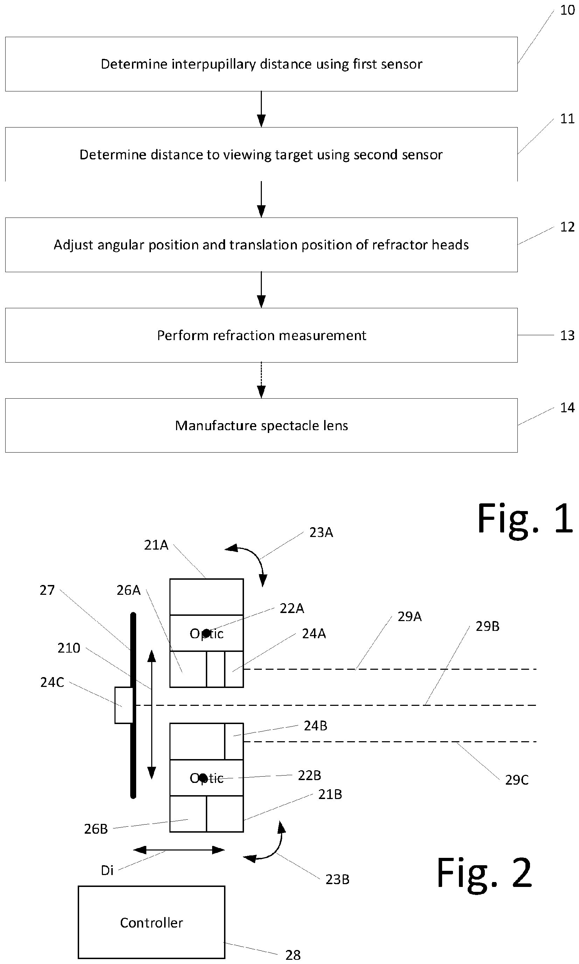

Fig. 1 describes a method according to an embodiment, and Fig. 2 shows a refractor in which the method of Fig. 1 may be performed.

The refractor of Fig. 2 includes a first refractor head 21A and a second refractor head 21B. First refractor head 21A is provided for a left eye of a patient and includes an optic 22A. Second refractor head 21B is provided for a right eye of a patient and includes an optic 22B. Optics 22A, 22B are conventional refractor head optics including an arrangement with variable refraction, holders for receiving variable lenses, elements for turning lenses to change the axis of refraction etc., as known to skilled persons. The refractor furthermore includes reference elements against which the head of the patient rests in operation. As an example, a plate 27 against which the front head of the patient is to abut during operation is shown in Fig. 2. As these are conventional elements so far, they will not be described in further detail.

Turning to Fig. 1, at step 10 the method comprising this determining an interpupillary distance (PD) of a patient using a second sensor. In the example of Fig. 2, a first camera 26A provided in first refractor head 21A and a second camera 26B provided in second refractor head 24B serve as second sensor. In other embodiments, the camera may also be provided at other locations. As explained above, the interpupillary distance of the patient may be determined in any conventional manner.

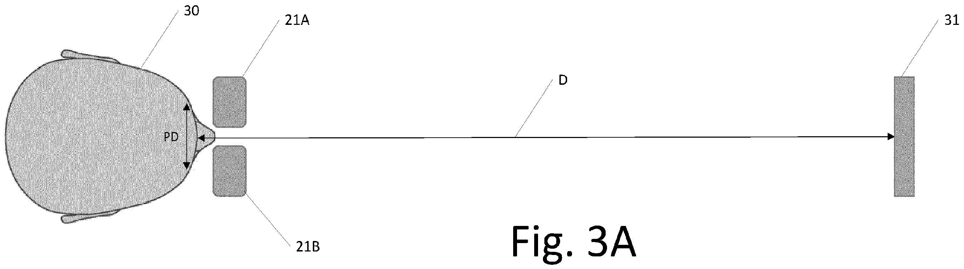

Returning to Fig. 1, at step 11 the method comprises determining a distance to a viewing target using a first sensor. Several examples for placements of first sensors are shown in Fig. 2. The first sensor may include a first sensor 24A arranged in first refractor head 21A, a first sensor 24B provided in second refractor head 21B, a first sensor 24C provided in element 27, or all of them. If more than one sensor is used, the distance may be determined by averaging the results of multiple sensors. Collectively, these will be referred to as first sensor 24 herein. As mentioned above, various sensor types like laser sensors, ultrasonic sensors or time of flight sensors may be used. Sensor 24 measures a distance in a direction indicated by dashed lines 29A, 29B, 29C to a viewing target (not shown in Fig. 2, but shown later in Figures 3A to 3C). The direction of dashed lines 29A, 29B and 29C is referred to as first direction herein. In this way, a distance between a head of a person, for example an eye portion, may be determined. In this respect, the sensor itself, as also explained above, may measure only an (unknown) part of this distance, and another part of the distance may be given by the geometry of the refractor. For example, when using the refractor of Fig. 2, as explained a forehead of the person in proper use abuts against element 27, such that the position of element 27 essentially coincides with the position of the head of the person. For example, with first sensor 24C the distance between the head of the person and the viewing target may be directly measured, as first sensor 24C is located essentially at the head of the person, when the forehead of the person abuts against element 27. In case first sensor 24A or first sensor 24B is used, a distance between the respective refractor head 21A or 21B and the viewing target is determined. This is an example for determining part of the distance between the head and the viewing target. In this case, the respective refractor head serves as a reference point as mentioned above. A distance Di between the head of the person (as determined by element 27) and the refractor head is fixed, such that also in this case the distance between the viewing target and the head of the patient can be determined as a sum of Di and the distance measured by first sensor 24A or 24B.

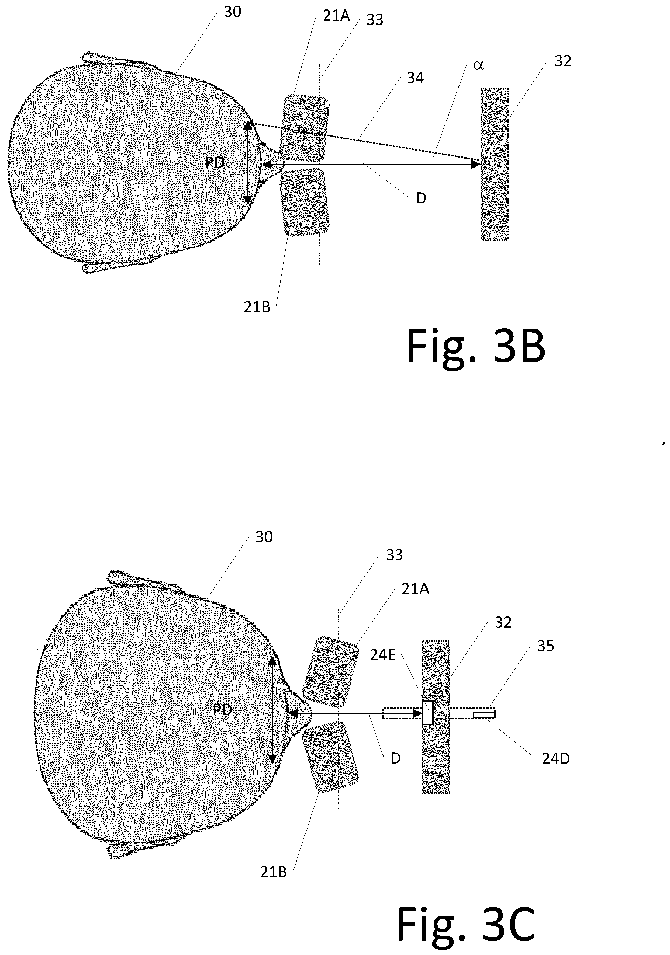

Further examples for sensor placements will be discussed further below referring to Fig. 3C.

Returning to Fig. 1, at step 12 then an angular position and a translation position of the refractor heads are adjusted based on the distance between the head and the viewing target and the interpupillary distance. The refractor heads 21A, 21B are movable independently from each other in a second direction as indicated by an arrow 210, and which is, in proper use, essentially parallel to a line between the pupils of the patient and perpendicular to the first direction. Furthermore, as indicated by an arrow 23A refractor head 21A is rotatable about an axis 22A and as indicated by an arrow 23B refractor head 21B is rotatable about an axis 22B. Axis 22A, 22B are perpendicular to the second direction and to the first direction as defined above. The adjusting at step 12 is controlled by a controller 28 in Fig. 2, i.e. controller 28 receives information from camera 26A, 26B or both and from first sensor 24 and then adjusts the translation position in the second direction and the angular position of refractor head 21A, 21B accordingly, such that the person essentially looks straight through optics 22A, 22B (for example along the optical axis of optics 22A, 22B, with possible tolerances) at the viewing target. As shown in Fig. 2, axes 22A, 22B may go through the optical axis of refractor heads 21A, 21B, although this need not be the case, as long as refractor heads 21A, 21B are movable in the second direction and rotatable about an axis perpendicular thereto.

Examples for this adjustment are now explained referring to Figures 3A to 3C. In each of Figures 3A to 3C, a head 30 of the patient is shown, together with refractor heads 21A and 21B explained with reference to Fig. 2 and a respective viewing target. PD in each of these Figures denotes the interpupillary distance, and D denotes the distance between head 30 and the respective viewing target.

In Fig. 3A, a far measurement is performed, for example with a distance D to a far viewing target 31 of more than 4m, for example 5m. In this case, the refractor heads 21A, 21B have an approximately parallel orientation, i.e. the optical axis of the optics of refractor heads 21A, 21B are approximately parallel to the first direction mentioned above. Approximately parallel means that for example if also here the angular position is determined based on the distance D, and D ist not infinity, a slight tilting of the refractor heads towards each other may remain. The adjustment in the second direction matches the pupillary distance PD, such that each eye looks straight through the optic of the respective refractor head 21A, 21B.

In Fig. 3B, a first example for a near measurement is shown. Here, a near viewing target 32, which may be different from far viewing target 31 (for example with respect to symbol size, text presented etc.) or may be the same, is provided at a significantly shorter distance than in Fig. 3A, for example at a distance of 40 cm. The interpupillary distance in this case may be for example 50mm, as in Fig. 3A. In this case, controller 28 controls the position in the second direction and the angular position of refractor heads 21A, 21B to take a convergence of the eye towards viewing target 32 into account, such that, as schematically indicated by a dotted line 34 for the left eye of patient 30, patient 30 still looks straight through the optics of refractor heads 21A, 21B. The rotation can be seen referring to a straight line 33.

Generally, an angle α of Fig. 3B can be calculated based on the interpupillary distance PD and the distance D, which are determined using the first and first sensors, according to tan α =PD/(2D).

Setting the correct translation positions along the second direction and angular positions of refractor heads 21A, 21B can then be easily done taking the geometry of the refractor, for example distance Di of Fig. 2, into account.

Fig. 3 shows a further example for a near measurement. Here, the near viewing target 32 is closer to head 30, for example at a distance of D=20cm. Additionally, the interpupillary distance PD in the example of Fig. 3C is larger than in Fig. 3B, for example 70mm. This, as can be seen in Fig. 3C, leads to a larger distance between refractor heads 21A, 21B in the second direction and a more pronounced inclination towards each other for the angular position.

Furthermore, Fig. 3C shows further examples for the placement of first sensor 24. These alternatives may also be applied to the previous embodiments.

The angular position may be adjustable continuously or in certain steps. A continuous adjustment is preferred as then a more precise adjustment to the respective interpupillary distance PD and distance D is possible.

In a first variation, near field target 32 is movable along a track 35 to change the distance D. In this case, a first sensor 24D may be provided which determines the position of near viewing target 32 on track 35, which in turn has a fixed spatial relationship to refractor head 21A, 21B. In this way, distance D can be determined. In this case, first sensor 24D is a sensor capable of sensing a translation movement of near viewing target 32 along track 35, for example a sensor based on a magnetic encoding or a sensor based on the control of a motor moving near viewing target 32. Alternatively, a first sensor 24E may be provided in viewing target 32, which again may be an ultrasonic sensor, laser sensor or time of flight sensor as already mentioned above, to measure the distance D to head 30 or a reference point (for example refractor head 21A or 21B) of the refractor.

Returning to Fig. 1, then at step 13 a refraction measurement is performed in a conventional manner, to determine a refraction of the patient. Based on the determined refraction, then at step 14 the refraction may be provided to a lens manufacturer, and a spectacle lens may be manufactured.

## Claims

1. A refractor, comprising:
a first refractor head (21A) and a second refractor head (21B), which are rotatable about respective axes (22A, 22B), and
a controller(28) configured to adjust angular positions of the first and second refractor heads (21A, 21B) about the respective axes (22A, 22B) on the basis of a distance (D) between a head (30) of the patient and a viewing target (31, 32) in a first direction perpendicular to the axes (22A, 22B),
**characterized by**
a first sensor (24A - 24E) configured to measure at least part of the distance between the head (30) and the viewing target (31, 32).

2. The refractor of claim 1, **characterized in that** the first sensor (24A - 24E) is provided in at least one of the first refractor head (21A) or the second refractor head (21B).

3. The refractor of claim 1 or 2, **characterized in that** the first sensor (24A - 24E) is selected from the group consisting of an ultrasonic sensor, a laser sensor and a time of flight sensor.

4. The refractor of any one of claims 1 to 3, **characterized in that** the viewing target (31, 32) is movable along a track (35), and the first sensor (24D) is configured to capture a position of the viewing target (31, 32) along the track (35).

5. The refractor of any one of claims 1 to 4, **characterized in that** the at least part of the distance is the complete distance (D) between the head (30) and the viewing target (31, 32).

6. The refractor of any one of claims 1 to 4, **characterized in that** the distance (D) is computed based on the at least part of the distance and a further distance (Di) given by the geometry of the refractor.

7. The refractor of any one of claims 1 to 6, **characterized in that** the first refractor head (21A) and the second refractor head (21B) are movable in a second direction (210) perpendicular to the axes (22A, 22B) and perpendicular to the first direction, wherein the controller (28) is further configured to adjust a position of the first and second refractor heads (21A, 21B) in the second direction (210) based on an interpupillary distance of the patient and to adjust the angular position of the first and second refractor heads (21A, 21B) about the respective axes (22A, 22B) also based on the interpupillary distance (PD).

8. The refractor of any one of claims 1 to 7, **characterized by** further comprising a second sensor (26A, 26B) configured to determine an interpupillary distance (PD) of a patient

9. The refractor of claim 8, **characterized in that** the second sensor (26A, 26B) comprises a camera.

10. A method for controlling a refractor including a first refractor head (21A) and a second refractor head (21B), comprising:
adjusting angular positions of the first and second refractor heads (21A, 21B) about respective axes (22A, 22B) on the basis of a distance (D) between a head (30) of a patient and a viewing target (31, 32) in a first direction, **characterized by**
measuring at least part of the distance (D) between the head (30) of a patient and the viewing target (31, 32) using a first sensor.

11. The method of claim 10, **characterized by** further comprising adjusting a position of the first refractor head (21A) and the second refractor head (21B) in a second direction perpendicular to the first direction and perpendicular to the axes (22A, 22B) based on an interpupillary distance (PD) of the patient, wherein adjusting the angular position of the first and second refractor heads (21A, 21B) about the respective axes (22A, 22B) is further based on the interpupillary distance (PD).

12. The method of claim 11, **characterized by** measuring an interpupillary distance (PD) of a patient using a second sensor.

13. The method of any one of claims 10 to 12, **characterized by** further comprising determining a refraction of at least one eye of the patient using the refractor.

14. A method of manufacturing a spectacle lens, comprising:
determining a refraction of at least one eye of a patient using the refractor of any one of claims 1 to 9 or by the method of claim 13, and
manufacturing the spectacle lens according to the refraction.
